# EUROPEAN PATENT APPLICATION

(11) **EP 2 423 217 A1**
(43) Date of publication of application: **29.02.2012**
(21) Application number: 10075359.9
(22) Date of filing: 23.08.2010
(51) Int. Cl.: C07K 1/22, C07K 14/32, C12N 15/62

(54) **Peptide and protein affinity tag from mistic protein**

(71) Applicant: Forschungsverbund Berlin E.V., 12489 Berlin (DE)
(72) Inventor: Bordag, Natalie, 13437 Berlin (DE); Keller, Sandro, 67655 Kaiserslautern (DE)
(74) Representative: Boeckh, Tobias

(57) **Abstract**

The invention relates to a peptide and a protein affinity tag comprising said peptide, characterized in that reversible oligomerisation of said peptide is controlled by a pH switch. The peptide of the present invention enables a reversible and stable interaction between monomers of the peptide of sufficient strength to physically tether proteins. When used as a protein affinity tag for purification of protein the peptide enables additive-free elution in conditions compatible with protein function and structure. The peptide exhibits furthermore a moderate size, thus rendering cleavage of the tag unnecessary.

SEQUENCE LISTING

<110>  Forschungsverbund Berlin e.V.
       Bordag, Natalie
       Keller, Sandro

<120>  Peptide and protein affinity tag

<130>  XII 646/10

<160>  6

<170>  PatentIn version 3.5

<210>  1
<211>  32
<212>  PRT
<213>  Artificial Sequence

<220>
<223>  Synthetic peptide

<400>  1

Met Lys Val Thr Ser Glu Glu Lys Glu Gln Leu Ser Thr Ala Ile Asp
1               5                   10                  15

Arg Met Asn Glu Gly Leu Asp Ala Phe Ile Gln Leu Tyr Asn Glu Ser
            20                  25                  30

<210>  2
<211>  14
<212>  PRT
<213>  Artificial Sequence

<220>
<223>  Synthetic peptide

<400>  2

Ile Asp Arg Met Asn Glu Gly Leu Asp Ala Phe Ile Gln Leu
1               5                   10

<210>  3
<211>  28
<212>  PRT
<213>  Artificial Sequence

<220>
<223>  Synthetic peptide

<400>  3

Met Lys Val Thr Ser Glu Glu Lys Glu Gln Leu Ser Thr Ala Ile Asp
1               5                   10                  15

Arg Met Asn Glu Gly Leu Asp Ala Phe Ile Gln Leu
            20                  25

<210>  4
<211>  22
<212>  PRT
<213>  Artificial Sequence

<220>
<223>  Synthetic peptide

<400>  4

Leu Ser Thr Ala Ile Asp Arg Met Asn Glu Gly Leu Asp Ala Phe Ile
1               5                   10                  15

Gln Leu Tyr Asn Glu Ser
            20

<210>  5
<211>  30
<212>  PRT
<213>  Artificial Sequence

<220>
<223>  Synthetic peptide

<400>  5

Met Lys Val Thr Ser Glu Glu Lys Glu Gln Leu Ser Thr Ala Ile Asp
1               5                   10                  15

Arg Met Asn Glu Gly Leu Asp Ala Phe Ile Gln Leu Tyr Asn
            20                  25                  30

<210>  6
<211>  32
<212>  PRT
<213>  Artificial Sequence

<220>
<223>  Synthetic peptide

<400>  6

Met Lys Val Thr Ser His His Lys Glu Gln Leu Ser Thr Ala Ile Asp
1               5                   10                  15

Arg Met Asn Glu Gly Leu His Ala Phe Ile Gln Leu Tyr Asn His Ser
            20                  25                  30

## Description

The invention relates to a peptide and a protein affinity tag comprising said peptide, characterized in that reversible oligomerisation of said peptide is controlled by a pH switch. The peptide of the present invention enables a reversible and stable interaction between monomers of the peptide of sufficient strength to physically tether proteins. When used as a protein affinity tag for purification of protein the peptide enables additive-free elution in conditions compatible with protein function and structure. The peptide exhibits furthermore a moderate size, thus rendering cleavage of the tag unnecessary.

### BACKGROUND OF THE INVENTION

Protein interactions are of central importance for many processes in living systems and range in duration and strength from brief short term interactions to stable and robust binding. Such stable interactions provide the basis for various medical and industrial applications, such as binding protein targets with antibodies or for protein purification via affinity interactions.

An important development in the engineering of protein interactions is the development of protein affinity tags. Protein affinity tags are generally peptide sequences with known affinities for certain reagents, either protein or otherwise, that are appended to proteins of interest. Such protein affinity tags can be used for a variety of purposes regarding the targeted physical interaction of proteins, such as for purification of endogenous or recombinant protein from a crude mixture of proteins or other contaminants.

The attachment of protein tags to the protein of interest generally occurs via modification of the reading frame at the DNA level, whereby a DNA sequence coding for the tag is ligated, or introduced via recombination, in frame with the DNA sequence coding for the protein of interest. Expression of the modified DNA sequence in a living system leads to production of a fusion protein.

After expression, such tagged fusion proteins are generally captured via interaction between the tag and its binding partner, which is attached to a solid phase. Contaminants are removed by washing thus leaving the isolated fusion protein bound to the solid phase. After binding and washing the isolated protein then needs to be released, via either elution or cleavage, from its interaction partner.

Due to the strength of the interaction between tag and binding partner, harsh conditions are often required for elution. However, conditions that maintain protein structure and activity are important for downstream applications of the purified protein, especially for proteins such as enzymes or antibodies. The release of the strong and stable interaction between tag and binding partner therefore represents a significant challenge when designing a protocol for protein purification. Typically, ligands with stronger affinity to the binding partner are added to elute the target protein but require subsequent separation from the solution before downstream applications may be conducted. The target protein can alternatively be proteolitically cleaved, although such cleavage requires also the subsequent separation of protease and target protein.

Additional problems regarding protein tags can also arise due to the size of the tag, which at times may be larger than the protein to be purified. Such large tag sizes may lead to misfolding of the protein of interest, leading to a loss of enzymatic activity or severe interference with protein function.

There are a multitude of protein affinity tags known in the art that can be applied for the targeted interaction or capture of proteins. However, such fusion tags exhibit the following disadvantages:
The addition of chemical substances is often required for elution. Such substances may lead to a disruption of protein structure and be removed by other methods, such as dialysis or subsequent chromatography.

Protein tags known in the art can be of significant size, thus limiting their application when purifying small proteins. Large tags may require protease digestion in order to separate the tag from its bound partner.

The binding between the tag and interaction partner may be to some extent unspecific, so that potentially contaminating proteins are also included in the "purified" sample. Such impurities and contaminants must be removed via other techniques in subsequent purification proceedings.

Specific examples of tags known in the art include the His-Tag, which is either eluted at acidic pH or at neutral pH with Imidazol. Imidazol leads to disruption of ITC and CD measurements and must be removed via dialysis before subsequent analysis. Through such methods, sensitive proteins can at times be rendered useless. His-Tags can also lead to further folding events which subsequently hinder the target protein in functional analysis. Many background or contaminant proteins are also know to contain histidines and are therefore included in the "purified" fraction of a protein preparation. Therefore, the His-Tag purification protocol must be individually optimised for each protein purification.

Biotin-streptavidin interactions and variations thereof are eluted using biotin. Due to the size of this tag, the tag itself must often be removed from the target protein before subsequent analysis.

The MBP-tag is eluted using maltose. This tag must also be removed due to its large size.

The GST-tag is usually directly cleaved using a protease, after having been bound to the solid phase (affinity chromatography column). The target protein must subsequently be separated from the protease in solution.

Antibodies and antigens have also been commonly used as means for the purification of recombinant proteins. During elution the antibody-antigen interaction is usually disrupted through the addition of SDS or through reduction of pH value. Both of these methods for elution can lead to significant disruption or destruction of protein structure.

The present invention therefore relates to a protein affinity tag based on peptide fragments of the Mistic protein. Mistic proteins have been described previously, whereby "Mistic" is an acronym for Membrane Integrating Sequence for Translation of Integral membrane protein Constructs. The structure and sequence of Mistic has been published (Roosild et al., 2005 Science 307:1317).

The disclosure of US patent application 2006/0211087 describes compositions and methods using Mistic polypeptides for producing and/or isolating proteins, especially eukaryotic integral membrane proteins, in heterologous expression systems, such as prokaryotic expression systems like *E. coli.* The Mistic class of polypeptides associate with the membrane (for example a bacterial cell membrane) when expressed alone or as a chimera with a fusion partner. This facilitates enhanced expression and recombinant production of membrane proteins in heterologous expression systems. Amino acid sequences of, and nucleic acid sequences encoding, Mistic polypeptides and their variants (including, e.g., functional fragments) are disclosed in the prior art.

The prior art discloses only full length Mistic variants and does not relate to uses or applications as a reversible multimerizing protein fusion tag. The present invention relates to peptide fragments of the Mistic protein, in particular a peptide fragment called "MB peptide", which exhibits unique oligomerization properties, therefore enabling its preferred use as a protein affinity tag.

### SUMMARY OF THE INVENTION

In light of the prior art the technical problem underlying the present invention is to provide a peptide that is suitable for inducing tethering between substances in solution, particularly for use as a protein affinity tag, that overcomes the disadvantages of the prior art. The peptide of the present invention enables a reversible and stable interaction via oligomerization, in addition to monomerization in conditions compatible with protein function and structure, therefore maintaining a moderate size rendering cleavage of the tag unnecessary.

This problem is solved by the features of the independent claims. Preferred embodiments of the present invention are provided by the dependent claims.

The invention therefore relates to a peptide, characterized in that reversible oligomerisation of said peptide is controlled by a pH switch.

In a preferred embodiment the peptide of the present invention is characterized in that when in solution at a pH below a defined switching pH value the peptide forms a well-defined, reversible oligomer and when in solution at a pH above the defined switching pH value the peptide exists as a monomer.

In another preferred embodiment the peptide of the present invention is characterized in that when in solution at a pH above a defined switching pH value the peptide forms a well-defined reversible oligomer and when in solution at a pH below the defined switching pH value the peptide exists as a monomer.

In a preferred embodiment the peptide of the present invention is characterized in that, when in solution at a pH below a defined switching pH value and the peptide forms an oligomer, the switching pH value is any value below 8, preferably 5.4.

In another preferred embodiment the peptide of the present invention is characterized in that, when in solution at a pH above a defined switching pH value and the peptide forms an oligomer, the switching pH value is any value above 6.

In another embodiment the peptide of the present invention is characterized in that the oligomer is a dimer, trimer, pentamer or preferably a tetramer, or exhibits any other degree of oligomerisation.

In a preferred embodiment the peptide of the present invention is characterized in that the amino acid sequence comprises:
- the sequence according to SEQ ID NO: 1, SEQ ID NO: 2, SEQ ID NO: 3, SEQ ID NO: 4, SEQ ID NO: 5 and/or SEQ ID NO: 6.
- a sequence having at least 80% homology to one or more of the sequences according to a).

A further aspect of the present invention relates to a nucleic acid molecule, characterized in that the sequence of the nucleic acid molecule encodes the peptide according to any one of the preceding claims.

A further aspect of the present invention relates to a nucleic acid molecule encoding a protein of interest, characterized in that the nucleic acid molecule that encodes the peptide of the present invention is attached in frame to the nucleic acid that encodes the protein of interest.

A further aspect of the present invention relates to a vector comprising the nucleic acid molecule encoding a protein of interest and the peptide of the present invention.

A further aspect of the present invention relates to a cell comprising the nucleic molecule and/or vector encoding a protein of interest and the peptide of the present invention.

A further aspect of the present invention relates to a protein comprising the peptide of the present invention, characterized in that the peptide is covalently attached to the protein, preferably as a recombinant fusion protein or via disulfide linkages.

A further aspect of the present invention relates to a solid phase comprising the peptide of the present invention, characterized in that the solid phase is preferably an affinity chromatography media, preferably comprising sepharose, more preferably in the form of beads, magnetic beads and/or microparticles.

A further aspect of the present invention relates to an affinity chromatography column comprising the solid phase as described above.

The invention further relates to use of the peptide of the present invention as a protein affinity tag, characterized in that the peptide is attached to a solid phase and independently attached to a protein of interest.

In a preferred embodiment the peptide of the present invention can be used as a protein affinity tag, whereby oligomerisation of the peptide induces binding between the peptide attached to the protein of interest and the peptide attached to the solid phase, and monomerization of the peptide prevents binding between the peptide attached to the protein of interest and the peptide attached to the solid phase.

A further aspect of the present invention relates to a protein affinity tag comprising the peptide of the present invention.

The present invention further relates to a method for the purification of protein, preferably recombinant protein, characterized in that
- the protein to be purified is covalently attached to the peptide of the present invention, preferably as a fusion protein,
- the peptide of the present invention is independently attached to a solid phase, preferably as described herein,
- the solid phase and the tagged protein to be purified are mixed in solution, whereby the pH of the solution induces and/or maintains oligomerisation of the peptide, and
- the tagged protein to be purified is eluted from the solid phase, whereby the pH of the elution induces monomerization of the peptide.

A further aspect of the invention relates to a method for the purification of protein, preferably recombinant protein, more preferably recombinant fusion protein, characterized in that
- the protein to be purified is covalently attached to the peptide of the present invention,
- oligomerisation of the peptide attached to the protein to be purified is induced by adjusting the pH, therefore inducing multimerization of the protein to be purified, and
- the protein to be purified is separated from contaminants via difference in molecular weight, preferably using dialysis, filtration, centrifugation and/or size-exclusion chromatography.

A further aspect of the present invention relates to a method for the purification of protein, whereby the protein to be purified is a membrane protein or an antibody.

A further aspect of the invention relates to use of the peptide of the present invention as a pH-indicator or a pH controlled switch, characterized in that
- Two or more substances, preferably enzyme and substrate, receptor and signaling molecule, pro-drug and activator, reactive chemicals A and B, and/or fluorescent donor and acceptor, to which the peptide of the present invention is independently attached, are provided,
- oligomerisation of the peptide attached to the substances is induced by adjusting the pH, therefore inducing multimerization of the substances, and
- the multimerization induces interaction or reaction between the substances, preferably as substrate turn over in the case of enzymes, receptor or signal cascade activation, pro-drug activation by cleavage, reaction between A and B, or distinct change in fluorescent activity.

The present invention further relates to a kit comprising the peptide of the present invention, the nucleic acid molecule and/or vector according encoding the peptide of the present invention, the cell according comprising the peptide of the present invention, the protein comprising the peptide of the present invention, the solid phase comprising the peptide of the present invention, the affinity chromatography column comprising the peptide of the present invention and/or the protein affinity tag comprising the peptide of the present invention, in addition to
- binding, wash and/or elution buffers, and/or
- means for covalent attachment of the peptide to a protein of interest and/or a solid phase.

### DETAILED DESCRIPTION OF THE INVENTION

The protein affinity tag of the present invention is distinguished from other tags known in the prior art on the basis of multiple physical and functional properties. The protein affinity tag described herein is capable of reversible oligomerization, which is dependent on the pH of the solution. In a preferred embodiment acidic pH induces oligomerization (preferably tetramerization) of the tag. The change in pH leading to induction of monomerization occurs in a sharp transition, thus allowing precise control of the oligomer state of the tag.

The pH value at which the induction (or prevention) of oligomerization is termed the "pH switching value". The phenomenon of pH-dependent structural reformation is termed a "pH switch". Importantly, the pH-induced oligomerization does not relate to simple aggregation of the protein affinity tag, as is common during aggregation or precipitation of proteins when exposed to extreme pH or in high protein concentrations. The pH-switch of the present invention enables engineered control of the oligomer state of the peptide at high protein concentrations whilst remaining soluble. Specific and reversible protein-protein interaction between monomers of the protein affinity tag can therefore be induced in solution.

The longest version of the MB peptide comprises of 32 amino acids and exhibits a molecular weight of 3.8 kDa and the sequence listed in SEQ ID NO: 1. Solubility of the peptide is good up until 2.2 mM (solubility experiments were carried out in phosphate buffer, using 0-600 mM NaF pH 6.5-9, or 154 mM NaF citrate buffer pH 4-7.8, or 20 mM NaCl Tris pH 8). Truncation experiments revealed that truncated versions of the 32 amino acid peptide also exhibit the pH-switch oligomerization phenomena and can be applied in the various indications of the present invention.

Table 1 discloses the preferred sequences of the present invention.

**Table 1. MB peptide and fragments thereof**

| **SEQ ID NO.** | **Description** | **Amino Acid Sequence** |
|---|---|---|
| SEQ ID NO: 1 | aa 1-32 | MKVTSEEKEQLSTAIDRMNEGLDAFIQLYNES |
| SEQ ID NO: 2 | aa 11-28 | IDRMNEGLDAFIQL |
| SEQ ID NO: 3 | aa 1-28 | MKVTSEEKEQLSTAIDRMNEGLDAFIQL |
| SEQ ID NO: 4 | aa 11-32 | LSTAIDRMNEGLDAFIQLYNES |
| SEQ ID NO: 5 | aa 1-30 | MKVTSEEKEQLSTAIDRMNEGLDAFIQLYN |
| SEQ ID NO: 6 | aa 1-32 | MKVTSHHKEQLSTAIDRMNEGLHAFlQLYNHS |

Sequence variants of the claimed proteins that maintain the said properties of the invention are also included in the scope of the invention. Protein modifications which occur through substitutions are also included within the scope of the invention. Substitutions as defined herein are modifications made to the nucleic acid or amino acid sequence of the protein, producing a protein which contains a different amino acid sequence than the primary protein without significantly altering the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, AIa, VaI, Ile and Leu; the non-polar aromatic amino acids Phe, Trp and Tyr; the neutral polar amino acids Ser, Thr, Cys, Gln, Asn and Met; the negatively charged amino acids Lys, Arg and His; the positively charged amino acids Asp and Glu, represent groups of conservative amino acids. This list is not exhaustive. For example, it is well known that Ala, Gly, Ser and sometimes Cys can substitute for each other even though they belong to different groups.

Conservative amino acid substitutions are not limited to naturally occurring amino acids, but also include synthetic amino acids. Commonly used synthetic amino acids are omega amino acids of various chain lengths and cyclohexyl alanine which are neutral non-polar analogs; citulline and methionine sulfoxide which are neutral non-polar analogs, phenylglycine which is an aromatic neutral analog; cysteic acid which is a positively charged analog and ornithine which is a negatively charged amino acid analog. Like the naturally occurring amino acids, this list is not exhaustive, but merely exemplary of the substitutions that are well known in the art.

Protein modifications or mutations may also occur through deletions. Deletions as defined herein are modifications made to the nucleic acid or amino acid sequence of the protein which produce a protein containing at least one amino acid less than the primary amino acid sequence of the protein, without significantly altering the function, preferably pH switchability.

The MB monomer is mostly unfolded (~70% random coil), whereby the MB tetramer forms an α-helix. Tetramerisation is weak in buffers with a pH >6.0, although significantly increases in pH <5.4. This pH sensitive switchability is reversible and occurs completely and suddenly from pH 6.0 to 5.4. At concentrations of up to 50 µM MB peptide in phosphate buffer at pH 6.5, the peptide remains purely as monomer. At pH values of <5.4, the MB peptide at the same concentration converts completely into tetramer according to results obtained with AUC (analytical ultracentrifugation) and CD spectroscopy.

The tetramerization of MB peptide is however dependent on temperature and the salt concentration of the buffer. In general, the proportion of MB peptide forming a tetramer is reduced with increasing temperature. At approximately 70°C, only monomers of the MB peptide are present. As the salt concentration increases, the tetramerisation increases accordingly at neutral pH values.

Various applications can be envisioned for the tetramerisation of the MB peptide tag of the present invention, for example the purification of target proteins tagged with the peptide, specific clustering and physical interaction of assemblies of enzymes tagged with the peptide or for pH regulated FRET experiments between molecules tagged with the peptide. Possible uses relate to the tethering or induction of interaction between any given two or more substances, such as enzyme and substrate, receptor and signalling molecule, pro-drug and activator, reactive chemicals A and B and/or fluorescent donor and acceptor. The tethering or interaction of said substances induces substrate turn over in the case of enzymes, receptor or signal cascade activation, pro-drug activation by cleavage, reaction between A and B, or distinct change in fluorescent activity.

One advantage of the present invention in the case of protein purification is that the protein to be purified can be eluted directly in the desired working buffer at neutral pH. This represents a significant simplification of existing protein purification procedures, whereby the usually required steps of washing and dialysis into new buffer conditions are avoided. Such a protein purification method represents a significant improvement over those methods known in the art, considering that the structural and functional integrity of the protein is maintained, in addition to saving time and cost. With a molecular model we demonstrate that the structural folding of the MB peptide occurs in a specific helical manner and does not lead to aggregation. This provides an enormous benefit over many purification strategies in that unwanted aggregation does not occur in addition to very minimal interaction with the target protein. A further advantage of the present invention is that the solubility of the MB peptide is very high and can in fact have a positive effect on target proteins of poor solubility.

One potential complication is that the MB protein tag could tetramerize with other tagged proteins in solution instead of tetramerizing to the MB peptide attached to the solid phase. However, this should not occur in significant amounts, considering that binding of the MB peptide fusion tag to a covalently immobilised MB peptide (attached for example to the affinity chromatography column) will always be stronger than interactions between freely diffusing interaction partners (as demonstrated via SPOT tests). A steric blocking of the tetramerisation between fusion tag MB peptide and the MB peptide of the column is also highly unlikely, considering the results of the SPOT tests. Additionally, it has been demonstrated that the transition from monomer to tetramer occurs via transiently passing through a dimer intermediate (AUC ultracentrifugation results). Such a dimer is also sufficient for molecule tethering purposes.

The "MB" peptide (mistic helix B) is intended to be applied as a protein fusion tag for the purification of recombinant proteins. The underlying principle of the purification scheme is the pH-sensitive structural modification, especially the specific and reversible tetramerisation of the MB peptide in response to changing from neutral to acidic pH-values (< 5.4). After successful fusion of the peptide to the target protein at the DNA-level the target protein is expressed using standard methods known in the art. The target protein is subsequently immobilised at acidic pH-values to a solid phase, preferably an affinity chromatography column, after destruction of the cell used for recombinant protein expression. The target protein is bound specifically via MB peptide oligomerization with MB peptide that has been independently covalently bound to a solid phase (of an affinity chromatography column). Contaminant proteins are subsequently washed from the column according to standard procedures using acidic buffers. Afterwards, the purified and immobilised target protein is eluted from the solid phase of the column by elution in buffer of neutral pH.

The present invention relates further to sequences that have been modified from the specifically listed sequences according to SEQ ID NO: 1, SEQ ID NO: 2, SEQ ID NO: 3, SEQ ID NO: 4 and/or SEQ ID NO: 5. The substitution of conserved amino acids is an obvious modification for one skilled in the art and thus falls under the scope of the present invention. Furthermore, as has been demonstrated in the examples of the invention, exchange of certain amino acids throughout the peptide can have little or no effect on the properties of the peptide itself.

However, the exchange of other amino acids can be used to modify the pH switching value of the peptide, so that the pH of the buffer used for the purification or assembly of any given protein or protein complex may be engineered precisely. This provides significant advantages over other affinity tags known in the prior art, which do not have the flexibility to be modified specifically according the preferred buffer pH conditions for any given target protein of interest. If a protein to be purified relies on a certain pH for structure or function, the switching value can be engineered to suit the protein of interest and thus facilitate a purification protocol without undue stress on the protein.

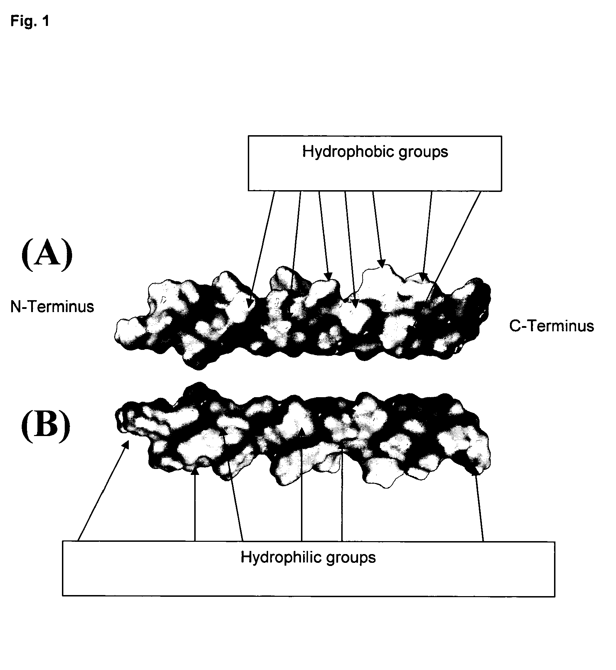

The amino acids E6, E7, D23 and E31 control the switch of the peptide between monomer and oligomer states. These particular pH switch relevant amino acids are adjacent to the hydrophobic interface and prevent oligomerization by electrostatic repulsion when charged (see Fig. 1). Modifications of these amino acids lead to changes in the pH switch value. The modification of all four amino acids (E6, E7, D23 and E31) to D residues leads to an increase in pH switch value of approximately 0.4. The replacement of D23 in E leads to a slight reduction in the pH switch value to approximately 5.5-6.0. The replacement of all four residues as histidine residues (SEQ ID NO: 6) reverses the switch properties of the peptide (when in solution at a pH below the defined switching pH value the peptide exists as a monomer), although maintains a similar pH switch value. Exchange of all four relevant amino acids to lysine also reverses the switch properties and additionally leads to a change of the switch value to pH >9 or 10. The exchange of all four relevant amino acids to arginine similarly reverses the switch properties and additionally leads to a change of the switch value to pH >11.

### FIGURES

- Fig. 1:: Molecular model of the secondary protein structure of the helical MB peptide.
- Fig. 2:: CD spectroscopy of MB at different pH values.
- Fig. 3:: Regeneration and reproducibility of the protein affinity tag.
- Fig. 4:: Mutation analysis of the amino acids important in oligomerization and pH switch.
- Fig. 5:: Summary of the single-point mutation and length analysis of the MB oligomerization.

### Detailed description of the figures

Figure 1: Molecular model of the secondary protein structure of the helical MB peptide. Hydrophobicity of the peptide MB is also indicated by differential after folding into an ideal helix. Hydrophilic groups are indicated with arrows from above, hydrophobic groups from below. (A) The hydrophobic areas in which the binding takes place. (B) View of the backside.

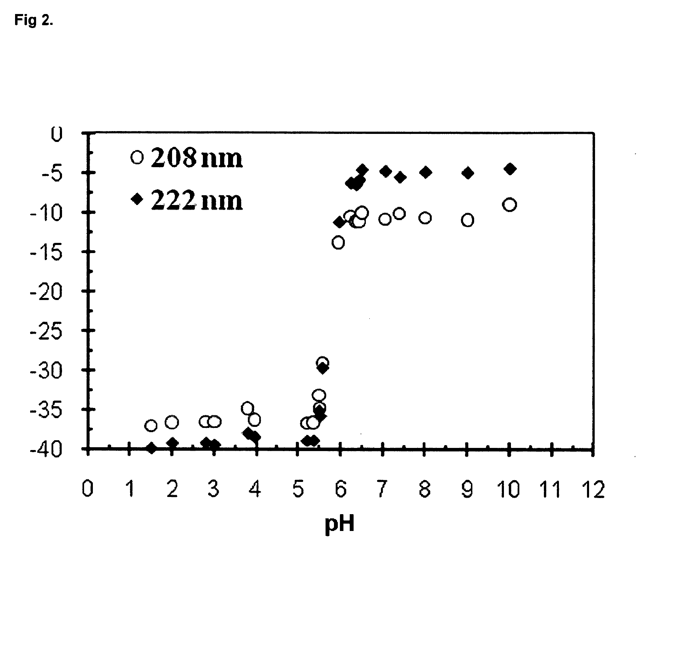

Figure 2: CD spectroscopy of MB at different pH values. Circular ellipticity of 50 µM MB at 208 nm or 222 nm was measured against buffer pH in 10 mM citrate or 10 mM phosphate buffer with 154 mM NaF at 20°C. Citrate buffer was applied for the pH range from pH 6.5 to 4 and phosphate buffer for the pH ranges 1.5 to 4 and 6.5 to 10. MB structure was uninfluenced by buffer type, because CD spectra in citrate buffer were equal to spectra in phosphate buffer in the overlapping pH regions. The wavelengths 208 nm and 222 nm are characteristic for α-helices. The ratio 208 nm / 222 nm decreased from 2.18 for pH>6 to 0.93 for pH<5.4.

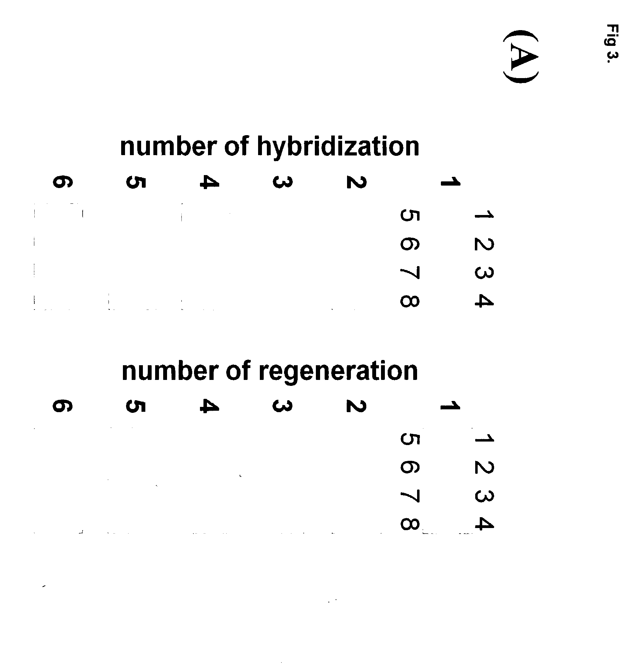

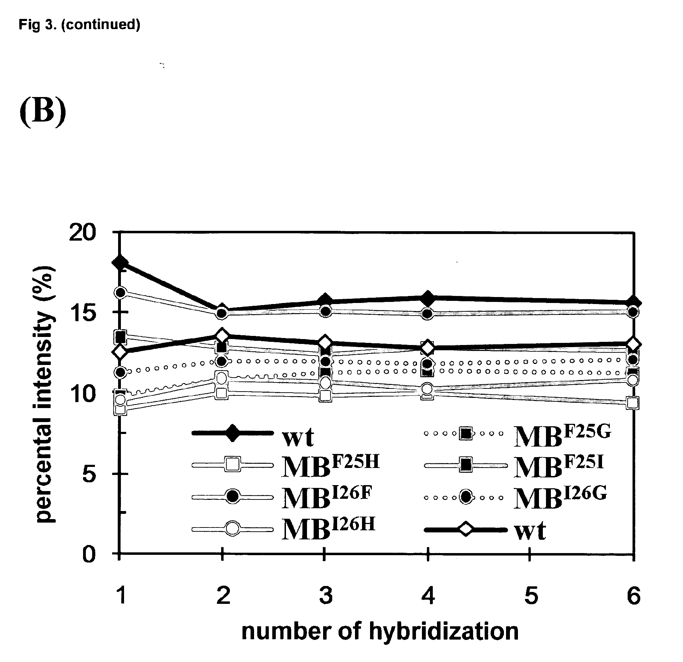

Figure 3: Regeneration and reproducibility of the protein affinity tag. (A) The snapshots of the same membrane repeatedly hybridised (left) and regenerated (right) show similar results after each of the six repetitions. All hybridisations were performed with 25 µM MB in 114 mM phosphate buffer with 50 mM NaF at pH 2.8 over night at 4°C under agitation. (B) Plot of the normalized median intensities of each spot against number of incubations.

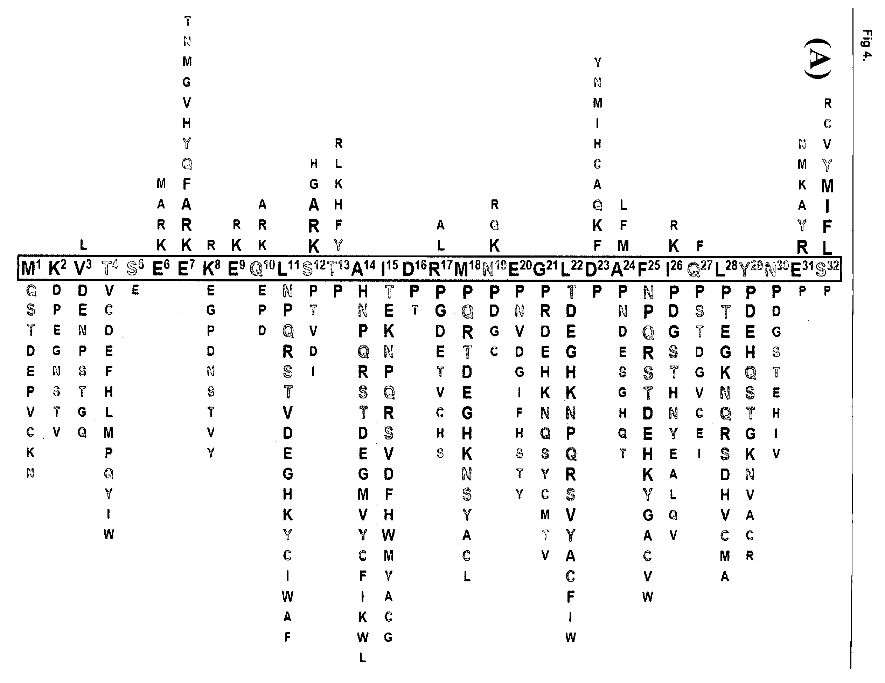

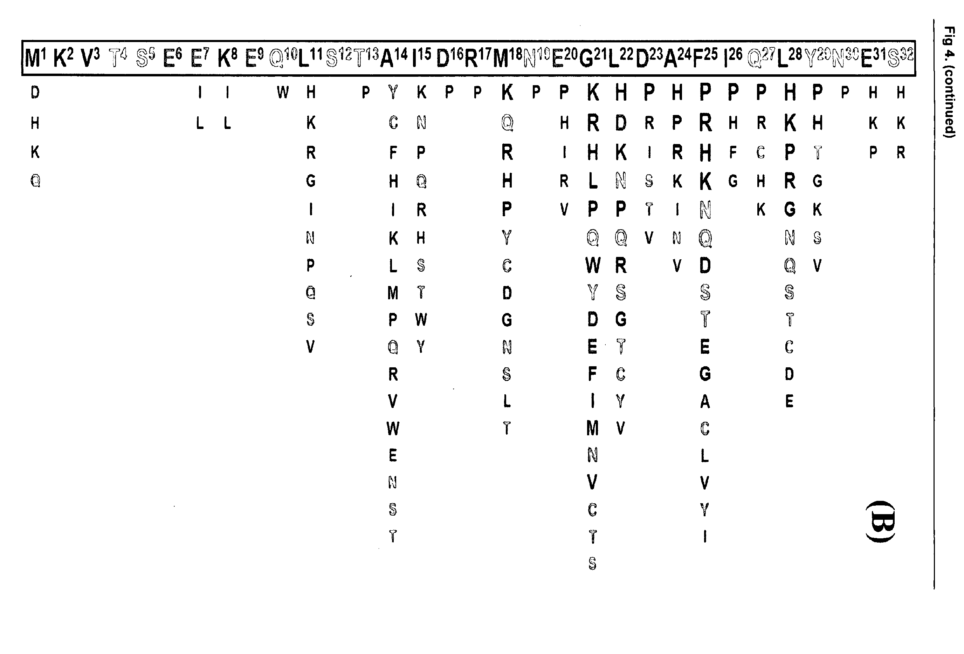

Figure 4: Mutation analysis of the amino acids important in oligomerization. Relevant single point mutations in (A) 10 mM phosphate buffer with 154 mM NaF at pH 6.5 or (B) 114 mM phosphate buffer with 50 mM NaF at pH 2.8. The amino acids numeration is shown as small numbers above the wild type letters, the wt sequence can be found in the middle (black frame). Letters listed vertically above the wt sequence intensify the interactions, letters below weaken or abolish. The bigger the letter size the stronger the influence, linear to the distance of the intensity to the wt mean intensity in times of SD, starting with a minimum of ±2 SD.

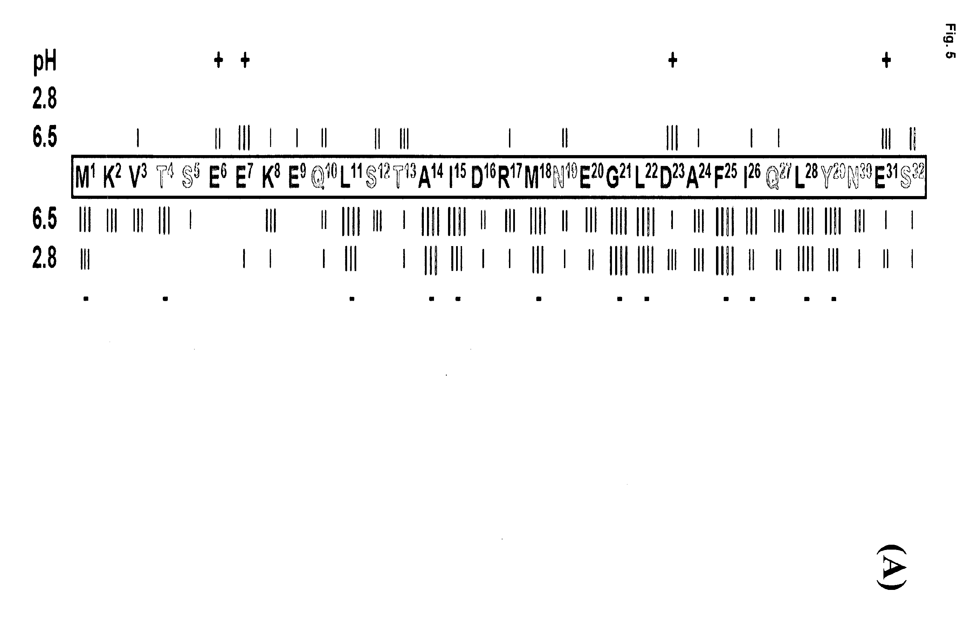

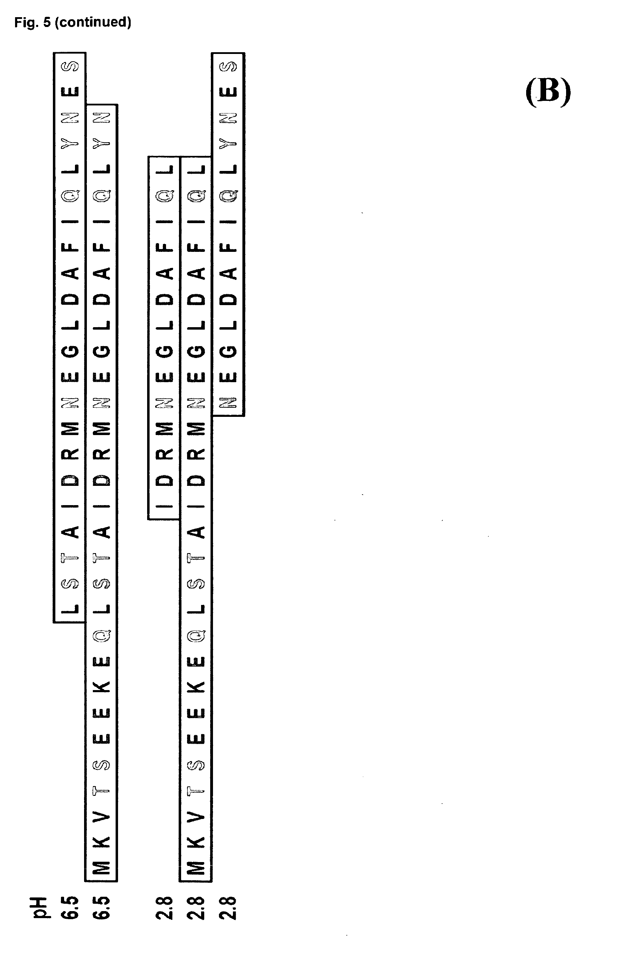

Figure 5: Summary of the single-point mutation and length analysis of the MB oligomerization (A) Single-point mutations substantially (p<0.05) influencing the oligomerization were grouped and are represented by bars above or below the wild type sequence (black frame).The amino acids numeration is shown as small numbers above the wild type letters. Bars above the wild type sequence signify an enhanced oligomerization and below a weakened or abolished. The bar size reflects the strength of the mutation's influences on the oligomerization. The buffer pH is given on the left side and hybridization were performed with either 75 µM MB in 10 mM phosphate buffer with 154 mM NaF at pH 6.5 or 25 µM MB in 114 mM phosphate buffer with 50 mM NaF at pH 2.8. Amino acid positions which were especially mutation-susceptible are marked by a + or -. (B) MB was systematically truncated with a step size of two amino acids from either one of the termini or simultaneously both termini. The shortest still with TAMRA-MB oligomerizing truncates are shown in black frames. The buffer pH is given on the left side and hybridization were performed with either 75 µM MB in 10 mM phosphate buffer with 154 mM NaF at pH 6.5 or 25 µM MB in 114 mM phosphate buffer with 50 mM NaF at pH 2.8.

### EXAMPLES

### Peptide selection

Four peptide fragments according to the four helices of Mistic were generated. The loops were split between the adjacent helices so that stringing together all four peptides would give the full-length Mistic. The present invention relates to one of these helices, named "MB peptide".

### Molecular Model

Previous results have shown that the MB peptide folds completely in an α-helical manner, in addition to the fact that the hydrophobic amino acids are especially important for the oligomerisation. Further, a molecular model of MB as an ideal α-helix was created using the SYBYL 8.0 software (Tripos International) to identify interaction surfaces. For this ideal α-helix model the the surface hydrophobicity was calculated with HINT (Virginia Commonwealth University and eduSoft, LC, Ashland, USA), using standard settings (see Fig. 1). From this analysis, a hydrophobic interface can be identified, over which the tetramerisation of the MB peptide occurs. The pH switch relevant amino acids are adjacent to the hydrophobic interface and prevent oligomerization by electrostatic repulsion when charged.

### Analysis of oligomerization properties

In order to assess the oligomerization properties of MB peptide circular dichroism spectroscopy (CD spectroscopy) was carried out according to standard procedures. The MB tetramerization was triggered by acidic pH values while remaining unchanged in basic conditions. Starting from basic pH 10 down to slightly acidic pH 6 the structure of MB was invariable at constant concentrations. Between pH 6 to 5.4 a rapid and tremendous increase in helicity occurred. Below pH 5.4 the pronounced α-helical MB structure remained constant down to pH 1.5 (see Fig. 2). The pronounced α-helical structure and structural invariance below pH 5.4 suggests that the tetramerization was complete.

MB peptide tetramerization was further characterized in acidic pH using AUC sedimentation velocity experiments according to standard procedures in 114 mM phosphate buffer with 50 mM NaF at pH 2.8 buffer. The results reconfirmed the above illustrated CD spectroscopic results. The type of homo-oligomerization remained unchanged and the tetramerization strength increased tremendously at acidic pH. Tetramerization appeared so strong that monomer content was too low for detection meaning that the K_{D} must be smaller than 1·10¹⁸ M³. Fitting of the results lead to a best result fit with a molecular mass of 15.2 kDa for the tetramer.

### SPOT synthesis, hybridization and analysis

A SPOT-analysis was carried out in order to assess oligomerization of the MB peptide and its ability to tether molecules. This approach is analogous to that of affinity chromatography, in that a solid phase-bound synthesized MB peptide is used to bind and isolate a molecule of interest that is tagged with the protein affinity tag of the present invention.

The nitrocellulose membrane which contained the MB peptide was incubated, and thus hybridized, with TAMRA-MB (MB peptide to which the TAMRA colouring agent has been covalently attached). If the spots remained coloured after washing an oligomerisation was judged to have taken place. Solid-phase bound MB peptide was synthesized directly on the membranes. Whatman 50 filter paper (Whatman GmbH, Dassel, Germany) was used for membrane-bound peptide synthesis. SPOT positions with a diameter of 3 mm were defined by treating isolated locations on the membrane with coupling reagents. The membrane bound peptides were assembled by the SPOT synthesizer MultiPep (Intavis Bioanalytical Instruments AG, Köln, Germany) according to standard protocols.

Spotted membranes were washed and blocked according to standard protocols before hybridization. After rinsing once with incubation buffer membranes were hybridized with TAMRA-MB over night at 4 °C under agitation. Incubation results were documented by scanning the membranes with a HP scanjet at 600 dpi.

### Regeneration of SPOT membranes and reversibility of Peptide MB Oligomerization

Hybridized membranes were thoroughly rinsed with washing buffer eluting bound TAMRA-MB at neutral pH. For quick removal 50% 2,2,2-trifluoroethanol/buffer at 50°C were utilized. Further incubations were carried out, subsequently washed and then hybridized again in order to test the reproducibility and reversibility of the MB peptide.

The excellent regeneration and reproducibility demonstrates one important aspect of the present invention, in that the binding is reversible and can be repeated a number of times using the same tag. The oligomerization was shown to occur successfully over six consecutive incubations and regenerations of eight spots yielding always reproducible results of the normalized spot intensities (see Fig. 3).

### Mutation scan for amino acids involved in oligomerization

In order to determine the various amino acid positions of the MB peptide that are important for oligomerization, amino acid substitutions were carried out at each position across the MB peptide. Each amino acid of the MB peptide was individually exchanged against all of the twenty naturally occurring amino acids. The influence of such amino acid exchanges was subsequently tested in regards to the oligomerisation strength of the peptide. The SPOT method is particularly well suited for such an assay, considering that different peptide sequences can be simultaneously synthesised on the surface of a nitrocellulose membrane. The synthesis of each specific peptide occurs in physically restricted areas, each of which is designated as a spot. Each spot therefore contains one exact sequence variant of the MB peptide.

The full mutation scans were either hybridized in neutral buffer (10 mM phosphate, 154 mM NaF, pH 6.5) or in acidic buffer (114 mM phosphate, 50 mM NaF, pH 2.8). Each hybridization was performed twice.

As can be seen from Figure 4 the tetramerization is characterized at both pHs by a helix pattern. Each helix turn is important, all 3 to 4 amino acids in such positions correspond with weaker or abolished interaction if exchanged. The C-terminus is more susceptible to modified oligomerization after amino acid exchange than is the N-terminus. Furthermore, many amino acids being part of classical transmembrane motifs are decisive in this tetramerization.

Results of the SPOT test are summarised in Fig. 5. The most important for oligomerization (and most common in transmembrane interactions) are the amino acids T4, L11, A14, I15, M18, G21, L22, F25, I26, L28, and Y29. Seven positions (E6, E7, S12, T13, D23, E31, S32) have been identified, which appear most important for potential enhancement of oligomerization. At neutral pH mutations into non-negatively charged amino acids intensifies interactions. Coincidentally, mutations in acidic pH at these positions, where the amino acids are fully protonated, show little or no effect at all.

The pH sensitivity appears to be based upon the amino acids: E6, E7, D23, and E31. The results of this test have also demonstrated that a complete solubilisation and rebinding from TAMRA-marked MB is possible. The elution process can be accelerated through application of warmth (approximately 50°C) and the addition of 50% trifluoroethanol.

The SPOT experiments further demonstrate that the amino acid sequence can be adjusted and modified whilst maintaining a specific oligomerisation. A shortening of the MB peptide can also lead to a modulation of the strength of the tetramerisation in addition to a reduction in potentially occurring interactions with target proteins. Through exchange of amino acids, the pH value at which the structural shift takes place can be modified. Additionally, further changes to the amino acid sequence may lead to changes in the oligomerisation, for example tetramerisation may be replaced by dimerization.

### Purification of recombinant GFP

The purification of recombinant GFP was carried out as an example of protein purification using the peptide tag of the present invention. The sequence according to SEQ ID NO 1 was cloned in frame to the GFP open reading frame in a suitable expression vector via ligation using standard cloning techniques. GFP was expressed in E. coli cultures according to standard methods. Cell lysis was performed by french press in buffer with pH 5, containing protease inhibitors and chelating agents. The filtered lysate was applied to an affinity column comprising peptide MB of the present invention attached to the solid phase using standard FPLC protocols and allowed to bind at low flow rate. The column was subsequently washed with binding (extraction) buffer of pH 5 until the baseline absorbance at 280 nm was reached. The isolated MB tagged GFP was eluted with elution buffer of pH 7. Subsequent SDS-PAGE analysis and silver staining revealed high protein purity of the tagged GFP.

### Purification of recombinant Proteinase K

To the Pro-Proteinase K an N-terminal tag according to a modified peptide MB was cloned in frame to the Proteinase K open reading frame using the same standard methods as described for GFP purification. The tagged Pro-Proteinase K was expressed in E. coli, inclusion bodies were extracted and subsequently solubilized as previously described (US 2007/0099283 A1). The solubilized inclusion bodies were mixed with magnetic beads to which the tag according to the present invention was covalently attached. The guanidine hydrochloride was diluted in buffer with a pH below the switching value, so that the tag oligomerization captures the tagged Pro-Proteinase K to the magnetic beads. Typical washing steps with buffer pH below 10.5 removed unbound protein and residual guanidine hydrochloride.

Renaturation was performed in folding buffer as described in (US 2007/0099283 A1). Autocleavage of the folded Pro-Proteinase K was started by addition of small amounts of catalytic Proteinase K. The cleaved and active Proteinase K binds non-covalently to the pro-peptide which itself is bound via the tag oligomerization to the magnetic beads. Elution of folded and active Proteinase K was achieved with a buffer pH of 10 containing small amounts of SDS, preferably 0.1 % (w/v). Thus the folded, active Proteinase K is removed from the pro-peptide which remains bound to the beads. The eluted Proteinase K buffer is then quickly titrated pH 8 - 10 to ensure Proteinase K stability. All steps were performed under soft agitation at 0-20 °C. The magnetic beads are regenerated by removing the tagged pro-peptide with 50% TFE at 50°C with a pH above 11.

## Claims

1. Peptide, **characterized in that**
reversible oligomerisation of said peptide is controlled by a pH switch.

2. Peptide according to the previous claim, **characterized in that**
when in solution at a pH below a defined switching pH value the peptide forms an oligomer and when in solution at a pH above the defined switching pH value the peptide exists as a monomer.

3. Peptide according to claim 1, **characterized in that**
when in solution at a pH above a defined switching pH value the peptide forms an oligomer and when in solution at a pH below the defined switching pH value the peptide exists as a monomer.

4. Peptide according to claims 1 or 2, **characterized in that**
the switching pH value is any value below 8, preferably 5.4.

5. Peptide according to claims 1 or 3, **characterized in that**
the switching pH value is any value above 6.

6. Peptide according to at least one of the preceding claims, **characterized in that** the amino acid sequence comprises:
- the sequence according to SEQ ID NO: 1, SEQ ID NO: 2, SEQ ID NO: 3, SEQ ID NO: 4, SEQ ID NO: 5 and/or SEQ ID NO: 6.
- a sequence having at least 80% homology to one or more of the sequences according to a).

7. Nucleic acid molecule, **characterized in that**
the sequence of the nucleic acid molecule encodes the peptide according to any one of the preceding claims.

8. Cell comprising the nucleic molecule according to claim 7.

9. Protein comprising the peptide according to at least one of claims 1 to 6, **characterized in that** the peptide is covalently attached to the protein, preferably as a recombinant fusion protein or via disulfide linkages.

10. Solid phase comprising the peptide according to at least one of claims 1 to 6, **characterized in that**
the solid phase is preferably an affinity chromatography media, preferably comprising sepharose, more preferably in the form of beads, magnetic beads and/or microparticles.

11. Affinity chromatography column comprising the solid phase according to claim 10.

12. Use of the peptide according to at least one of claims 1 to 6 as a protein affinity tag, **characterized in that**
the peptide is attached to a solid phase, preferably according to claim 10, and independently attached to a protein of interest.

13. Protein affinity tag comprising the peptide according to at least one of claims 1 to 6.

14. Method for the purification of protein, preferably recombinant protein, **characterized in that**
- the protein to be purified is covalently attached to the peptide of claims 1 to 6, preferably as a fusion protein,
- the peptide of claims 1 to 6 is independently attached to a solid phase, preferably according to claim 10,
- the solid phase and the tagged protein to be purified are mixed in solution, whereby the pH of the solution induces and/or maintains oligomerisation of the peptide, and
- the tagged protein to be purified is eluted from the solid phase, whereby the pH of the elution induces monomerization of the peptide.

15. Method for the purification of protein, preferably recombinant protein, more preferably recombinant fusion protein, **characterized in that**
- the protein to be purified is covalently attached to the peptide of claims 1 to 6,
- oligomerisation of the peptide attached to the protein to be purified is induced by adjusting the pH, therefore inducing multimerization of the protein to be purified, and
- the protein to be purified is separated from contaminants via difference in molecular weight, preferably using dialysis, filtration, centrifugation and/or size-exclusion chromatography.
